# EUROPEAN PATENT SPECIFICATION

(11) **EP 1 564 199 B1**
(45) Date of publication and mention of the grant of the patent: **27.04.2011**
(21) Application number: 02808059.6
(22) Date of filing: 24.10.2002
(51) Int. Cl.: C07C 15/04, C07C 4/12

(54) **METHOD FOR PRODUCING BENZENE FROM ALKYLBENZENE-CONTAINING MIXTURES HAVING A HIGH CONTENT OF SULPHUR-CONTAINING SUBSTANCES**
HERSTELLUNGSVERFAHREN FÜR BENZOL AUS ALKYLBENZOLHALTIGEN MISCHUNGEN MIT HOHEM GEHALT AN SCHWEFELHALTIGEN SUBSTANZEN
PROCEDE DE PRODUCTION DE BENZENE A PARTIR DE MELANGES CONTENANT DES ALKYLBENZENES A FORTE TENEUR EN SUBSTANCES CONTENANT DU SOUFRE

(43) Date of publication of application: 17.08.2005
(73) Proprietor: Naumenko, Aleksandr Olegovich, Moscow, 125212 (US)
(72) Inventor: NAUMENKO, Aleksandr Olegovich, Moscow, 125212 (RU); PANTUYEV, Mikhail Valerievich, Moscow, 103498 (RU)
(74) Representative: Einsel, Martin
(86) International application number: PCT/RU2002/000467
(87) International publication number: WO 2004/037755

(56) References cited:
- GB-A- 982 522
- US-A- 3 198 846
- US-A- 3 310 592
- US-A- 4 463 206
- DATABASE WPI Section Ch, Week 200314 Derwent Publications Ltd., London, GB; Class E14, AN 2003-146381 XP002349750 -& RU 2 193 548 C1 (KEIMVEST STOCK CO) 27 November 2002 (2002-11-27)
- BERTENTS A.D. ET AL.: 'Pererabotka zhidkikh produktov piroliza' KHIMIYA 1985, MOSCOW, pages 108 - 113, XP002999097
- SULIMOV A.D.: 'Proizvodstvo aromaticheskikh uglevorodorodov iz neftyanogo syriya' KHIMIYA 1975, pages 263 - 267, XP008053814

## Description

### SCOPE

This invention related to the petrochemical industry, and, in particular, to methods for producing benzene from various saturated alkylbenzene-containing mixtures produced in various processes.

### PRIOR ART

Known in the art arc various methods for producing benzenes from aromatic hydrocarbons, in particular toluene or alkyl naphthalene, comprising thermally hydrodealkylating the same (see, for example, SU 277,758, 1970; and SU 1,541,198, 1990). These prior art methods comprise producing benzene from mixtures purified from sulfur-containing aromatic hydrocarbons, and do not relate to the processing of highly aromatic fractions of liquid pyrolysis or coking products having a high content of sulfur-containing substances (in excess of 0.15% of sulfur).

Known in the art also are various methods of hydrodealkylation of alkyl-benzenes comprising recycle of un-dealkylated products.

According to the method as described in US 3198846 the total effluent of the hydrodesulfurization reactor is charged into the hydrodealkylation zone along with unreacted or partially reacted alkylated aromatics in order to obtain substantially sulfur-free aromatics.

According to the method of US 3310592 in the second step hydrodealkylation occur in the presence of small amounts of sulfur together with a recycle of un-dealkylated aromatics.

According to the method of GB 982522 hydrodealkylation of alkylaromatics is carried out in the presence of sulfur together with recycle of unreacted alkylaromatics.

These methods do not solve the problem of processing highly aromatic fractions with high sulfur content (from 0.15 to 1. mass%) in technological lines with two-stage catalytic hydrogenation, where catalyst can be spoiled by sulfur.

The closest prior art of the method of this invention is a method for producing benzene from benzene- and saturated alkylbenzene-containing mixtures produced by processing aromatic fractions of liquid pyrolysis products, comprising subjecting them to preliminary two-stage catalytic hydrogenation by thermally hydrodealkylating the aromatic compounds to benzene and returning part of the product for hydrodealkylating un-dealkylated products (see A.D. Borentz et al., "Processing of Liquid Pyrolysis Products," Chemistry, Moscow, 1985, pp. 88-97, 108-121). The prior art method is applicable to the processing of aromatic fractions having a relatively low sulfur content, because the product is completely purified from sulfur-containing substances before the catalytic hydrogenation stage (see: *ibid.* p. 111), but it does not use highly aromatic fractions of liquid pyrolysis products having a high content of sulfur-containing compounds, which contain an insignificant quantity of unsaturated compounds, as feedstock for the process.

### SUMMARY OF THE INVENTION

It is an object of this invention to develop a method for producing benzene, which comprises processing a highly aromatic fraction having a relatively high content of sulfur-containing compounds, bypassing two-stage catalytic hydrogenation.

The technical effect of this invention consists in processing a highly aromatic fraction of liquid pyrolysis or coking products, as well as processing aromatic fractions having a low sulfur content.

This technical effect is achieved in a method for producing benzene from benzene-and/or saturated alkylbenzene-containing mixtures, wherein said compounds are hydrodealkylated and the un-dealkylated component is returned to the hydrodealkylation stage for recycling, and the highly aromatic fraction with at least 60 mass % of C₆-C₁₅ aromatic compounds and with an elevated content of sulfur-containing substances from 0.15 to 1.5 mass % is added to the input of the hydrodealkylation reactor.

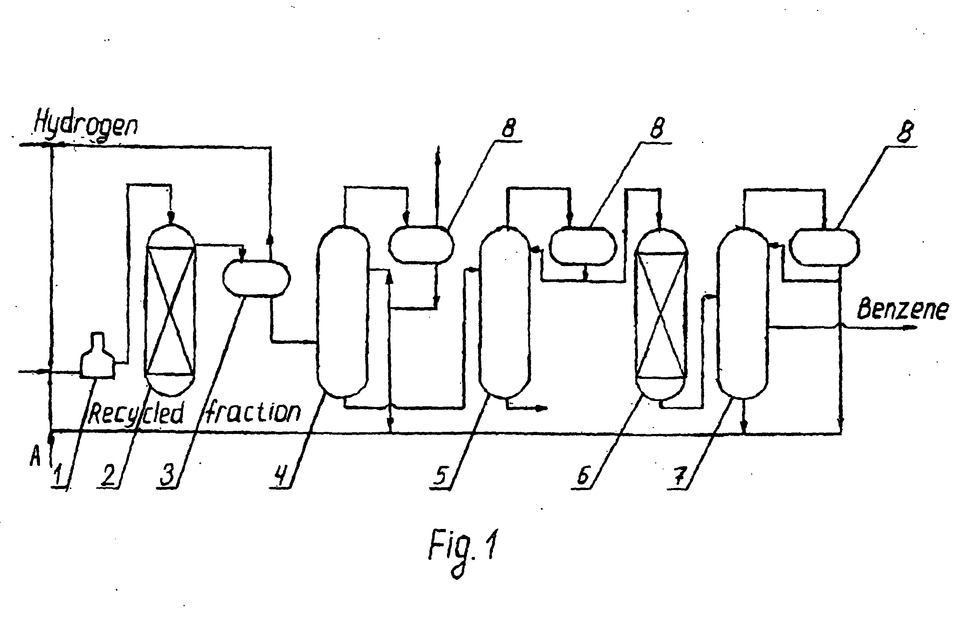

### DESCRIPTION OF THE DRAWING

The drawing shows a schematic diagram of a process line used for producing benzene, which comprises furnace 1, hydrodealkylation reactor 2, separator 3, stabilizing column 4, pre-fractionating column 5, catalytic after-purification reactor 6, benzene column 7, and vessels 8.

### DESCRIPTION OF THE PREFERRED EMBODIMENT OF THE INVENTION

The process line of equipment used for processing aromatic fractions of liquid pyrolysis and/or coking products having boiling temperatures ranging from 60°C to 340°C into benzene provides for two-stage hydrogenation of the products on palladium sulfide and aluminum-cobalt-molybdenum catalysts (not shown) in order to achieve complete hydrogenation of diene and unsaturated compounds and sulfur-containing compounds, followed by hydrocracking of the paraffin compounds and thermal hydrodealkylation of aromatic compounds, in particular, toluene, o-, m- and n-xylenes, ethylbenzene, and so on, to benzene in the hollow in-tandem mixing and displacement reactors 2 (only one reactor is shown in the drawing for convenience). The product then flows successively into the stabilizing column 4, pre-fractionating column 5, catalytic after-purification reactor 6, and benzene column 7.

The production process requires the product containing un-dealkylated aromatic compounds to be returned in part from the column 7, along a recycling line, to the output of the hydrodealkylation reactor 2.

The after-purification reactor 6 is used to achieve a more complete purification of the product.

A highly aromatic fraction of liquid pyrolysis and/or coking products having boiling temperatures ranging from 60°C to 340°C, containing at least 60 mass% of C₆-C₁₅ aromatic compounds and an elevated content of sulfur-organic compounds of not more than 1.5 mass% of sulfur, is processed by feeding it batch-wise (along arrow A in the drawing) into the recycling line of the recycled aromatic fraction fed into the hydrodealkylation reactor 2. If more sulfur is removed from the product, the quantity of the fraction fed into the recycling line is chosen so that the total content of sulfur-organic compounds at the output of the hydrodealkylation reactor 2 does not exceed an admissible level depending on the potentialities of the process line to remove more sulfur from the final product. The total content of sulfur-organic compounds does not exceed 0.1 mass% for the above-described production process.

A highly aromatic fraction can be fed into the recycled hydrodealkylation product because it contains an insignificant quantity of unsaturated compounds and can be processed bypassing the hydrogenation stage.

An embodiment of the method is illustrated by the following example:
Characteristics of the hydrodealkylation reactor:
Input: - liquid hydrolysis products are fed from the hydrogenation stage at a rate of 20 tons/hour;
- recycling, 2.5 tons/hour;
- highly aromatic fraction with an elevated sulfur content, 2.5 tons/hour

| | |
|---|---|
| Pressure: | 22 atm. |
| Temperature: | 600-700°C |
| H₂/feedstock ratio: | 1,000/1 m³/1m³ |
| Output: | sulfur content, 0.02 mass% |

Commercial benzene with a sulfur content < 0.5 ppm is obtained downstream of the stabilizing and pre-fractionating columns, and the after-purification benzene fractionating column.

The heavy fraction is recycled from the benzene fractionating column to the input of the dealkylation reactor.

Composition of the recycled stream:
< 1% benzene;
= 90% toluene;
= 8-9% total xylenes;
< 0.1% nonaromatic compounds.

### INDUSTRIAL APPLICABILITY

The method of this invention can be used in process lines to process aromatic fractions of liquid pyrolysis or coking products into benzene with the use of equipment currently installed in the line.

## Claims

1. A method for producing benzene from alkylbenzene-containing mixtures, comprising two-stage hydrogenation on palladium sulfide and aluminum-cobalt-molybclenum catalysts followed by hydrocracking of parraffin compounds and thermally hydrodealkylating is said compounds in a hollow in tandem mixing and displacement reacting (2) and recycling the un-dealkylated component to the hydrodealkylation stage, wherein a highly aromatic fraction of liquid pyrolysis and/or coking products having boiling temperatures ranging from 60°C to 340°C and with at least 60 mass% of C₆-C₁₅ aromatic compounds and having a content of sulfur-containing substances up to 1.5 mass% of sulfur is added batchwise to the recycled product bypassing hydrogenation stage, and feeding the recycled product, the highly aromatic fraction and the compounds of the hydrogenation stage to the hydrodealkylation stage, and feeding the product of the hydrodealkylation stage successively into a stabilizing column (4), a prefractionating column (5), a catalytic after-purification reactor (6) and benzene column (7) and returning un-dealkylated components in part from column (7), along a recycling line to the hydrodealkylating stage.

2. A method as set forth in claim 1, wherein the highly aromatic fraction is fed in a quantity such that the total content of sulfur-containing compounds at the output of the hydrodealkylation reactor does not exceed an admissible level depending on the potentialities of the process line for further desulfurization of the final product.

## Patentansprüche

1. Verfahren zur Herstellung von Benzol aus Alkylbenzol enthaltenen Mischungen, umfassend eine Zweistufenhydrierung mit Palladiumsulfid und Aluminium-Kobalt-Molybdänkatalysatoren mit anschließendem Hydrocracken der Paraffinverbindungen und thermischer Hydrodealkylierung der Verbindungen in hohlen in Tandem Vermischungs- und Verdrängungsreaktoren (2) und Rückführung der nicht dealkylierten Komponenten zu der Hydrodealkylierungsstufe, wobei eine hocharomatische Fraktion von flüssigen Pyrolyse- und/oder Verkokungsprodukten mit einer Siedetemperatur in einem Bereich von 60 °C bis 340 °C und mit mindestens 60 Masseprozent an C₆₋₁₅ aromatischen Verbindungen und mit einem Gehalt an Schwefel enthaltenen Substanzen mit bis zu 1,5 Masseprozent Schwefel batchweise dem rückgeführten Produkt unter Umgehung der Hydrierungsstufe zugesetzt wird,
Zugabe des rückgeführten Produkts, der hocharomatischen Fraktion und der Verbindungen aus der Hydrierungsstufe zur Hydrodealkylierungsstufe, und nacheinander Zugeben des Produktes der Hydrodealkylierungsstufe zu einer Stabilisierungssäule (4), einer Pre-Fraktionierungssäule (5), einem katalytischen Nachreinigungsreaktor (6) und einer Benzolsäule (7), und teilweise Rückführung der nicht dealkylierten Verbindungen aus der Säule (7) entlang einer Rückführungsstrecke zu der Hydrodealkylierungsstufe.

2. Verfahren nach Anspruch 1,
wobei die hocharomatische Fraktion in einer Menge zugesetzt wird, so dass der Gesamtgehalt an schwefelhaltigen Verbindungen am Ausgang des Hydrodealkylierungsreaktors einen zulässigen Level nicht übersteigt, der von dem Vermögen der Prozessstrecke zur weiteren Entschwefelung des Endproduktes abhängt.

## Revendications

1. Procédé de production de benzène à partir de mélanges contenant des alkylbenzènes, comprenant une hydrogénation sur sulfure de palladium en deux stades et des catalyseurs aluminium-cobalt-molybdène puis un hydrocraquage des composés de paraffine et une hydro-désalkylation thermique desdits composés dans des réacteurs de mélange et de déplacement en tandem creux (2) et le recyclage du composant non désalkylé au stade d'hydrodésalkylation, dans lequel une fraction hautement aromatique de pyrolyse liquide et/ou des produits de cokéfaction ayant des températures d'ébullition de 60° C à 340° C et comprenant au moins 60 % en poids de composés aromatiques en C₆ à C₁₅ et ayant une teneur en substances contenant du soufre allant jusqu'à 1,5% en poids de soufre est ajoutée par lot au produit recyclé en évitant le stade d'hydrogénation,
l'acheminement du produit recyclé, de la fraction hautement aromatique et des composés du stade d'hydrogénation au stade d'hydrodésalkylation et
l'acheminement du produit du stade d'hydrodésalkylation successivement dans une colonne de stabilisation (4), une colonne de préfractionnement (5) un réacteur catalytique après purification (6) et une colonne de benzène (7), et
le retour des composants non désalkylés en partie de la colonne (7) le long d'un circuit de recyclage au stade d'hydrodésalkylation.

2. Procédé selon la revendication 1, dans lequel la fraction hautement aromatique est acheminée en une quantité telle que la teneur totale des composés contenant du soufre à la sortie du réacteur d'hydrodésalkylation ne dépasse pas un niveau admissible en fonction des possibilités du circuit de traitement pour désulfuriser encore le produit final.
